(19) Europäisches Patentamt
European Patent Office
Office européen des brevets

(11) **EP 0 514 785 B1**

(12) **EUROPÄISCHE PATENTSCHRIFT**

(45) Veröffentlichungstag und Bekanntmachung des Hinweises auf die Patenterteilung:
**22.01.1997 Patentblatt 1997/04**

(51) Int Cl.$^6$: **C07H 15/203**, C07H 15/18

(21) Anmeldenummer: **92108234.3**

(22) Anmeldetag: **15.05.1992**

(54) **Alkyl-substituierte Aryl-Saccharide, oberflächenaktive Mittel, die solche Saccharide enthalten und ihre Verwendung in diagnostischen Tests**

Alkyl substituted aryl-saccharides, surfactants containing such saccharides, and their use in diagnostic tests

Aryl-saccharides alcoyl-substitué, agents tensio-actifs les contenants, et leur utilisation en test diagnostique

(84) Benannte Vertragsstaaten:
**AT BE CH DE DK ES FR GB GR IT LI LU NL PT SE**

(30) Priorität: **17.05.1991 DE 4116257**

(43) Veröffentlichungstag der Anmeldung:
**25.11.1992 Patentblatt 1992/48**

(73) Patentinhaber: **BOEHRINGER MANNHEIM GMBH
68298 Mannheim (DE)**

(72) Erfinder:
- **Sluka, Peter, Dr.
  W-8120 Weilheim (DE)**
- **Batz, Hans-Georg, Dr.
  W-8132 Tutzing (DE)**
- **Vogt, Bernd, Dr.
  W-8132 Tutzing (DE)**

(74) Vertreter: **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08 20
81635 München (DE)**

(56) Entgegenhaltungen:
- **LIQUID CRYSTALS Bd. 8, Nr. 6, 1990, LONDON GB Seiten 885 - 888 TSCHIERSKE C. ET AL 'Amphiphilic carbohydrate-based mesogens incorporating structal feature of calamitic liquid crystals'**
- **DATABASE WPIL Section Ch, Week 9202, Derwent Publications Ltd., London, GB; Class D13, AN 92-012737**
- **BIOCHEMISTRY. Bd. 25, 1986, EASTON, PA US Seiten 2522 - 2529 DALE M.P. ET AL '. beta.-glucosidase: Substrate, Solvent, and Viscosity Variation as Probes of the Rate-Limiting Steps'**
- **CARBOHYDRATE RESEARCH. Bd. 142, 1985, AMSTERDAM NL Seiten 333 - 337 KLEINE H.P. ET AL 'Phase-transfer-catalyzed synthesis of 2,3 ,4,6-tetra-O-acetyl-.beta.-D- galactopyrano sides'**

**Beschreibung**

Die Erfindung betrifft die Verwendung von Arylsacchariden als oberflächenaktive Mittel in diagnostischen Tests.

Die Auswertung von diagnostischen Tests wird häufig durch hydrophobe Bestandteile, die über zu analysierende Körperflüssigkeiten, wie beispielsweise Serum, in das Testsystem gelangen, beträchtlich gestört. Die häufigsten solcher auftretenden Störungen sind z.B. Trübungen im Testsystem oder auch unspezifische Wechselwirkungen von hydrophoben Bestandteilen aus der Probe oder den Reagenzien mit anderen Komponenten des Tests. Es ist daher bereits versucht worden, zur Vermeidung dieser Störungen nicht-ionische Tenside vom Typ der Alkyl- und Arylethoxylate als Hilfsmittel in diagnostischen Tests einzusetzen. So ist es z.B. aus der DE-OS 28 04 356 bekannt, Alkylphenoxypolyethoxyethanol mit einer Polyoxyethylenkette von weniger als 20 Oxyethyleneinheiten als oberflächenaktive Substanz bei der Bestimmung von Cholesterinestern zu verwenden, die proteingebunden im Blutserum vorliegen.

Aus der WO88/07683 ist es bekannt, in Enzymimmunoassays, die Peroxydasekonjugate enthalten, ein Detergenz aus einem oder mehreren Polyoxyethylenethern und zwar insbesondere Triton® X-100 zuzusetzen und damit die Empfindlichkeit des Tests zu erhöhen.

Die bislang zur Beseitigung der beschriebenen Störung in diagnostischen Testsystemen verwendeten Tenside sind jedoch ausschließlich technische Produkte, die durch die Anlagerung von Ethylenoxid an die entsprechenden Alkohole bzw. Phenole gewonnen werden. Dieser Herstellungsprozeß bedingt jedoch eine starke Schwankungsbreite der Ethoxylierungsgrade und der Qualität der Produkte. Darüber hinaus enthalten diese Substanzen je nach Charge in unterschiedlichen Mengen Verunreinigungen wie Peroxide, Carbonylverbindungen und Schwermetallsalze. Alle diese mit dem Tensid eingeschleppten Verunreinigungen können bei der Verwendung solcher Testsysteme zu unerwünschten Wirkungen und damit zu fehlerhaften Ergebnissen führen.

Schließlich gelten ethoxylierte i-Octylphenole auch als biologisch schlecht abbaubar und können daher in Kläranlagen nicht entsorgt werden. Sie sollten deshalb aus Umweltschutzgründen nicht in das Abwasser gelangen.

Die Erfindung hat daher zum Ziel, ein nicht-ionisches Tensid bzw. ein oberflächenaktives Mittel bereitzustellen, das in diagnostischen Tests durch hydrophobe Bestandteile auftretende Störungen beseitigt oder vermindert und das die zuvor geschilderten Nachteile nicht aufweist.

Überraschenderweise wurde nun gefunden, daß sich dieses Ziel erreichen läßt durch Verwendung von Verbindungen der allgemeinen Formel

$$S - Ar - X$$

worin

Ar ein Benzyl- oder Phenylrest,
S ein mit Ar über eine $\alpha$- oder ß-glycosidische Verbindung verknüpfter Saccharidrest und
X ein geradkettiger oder verzweigter Alkylrest mit 2 bis 20 Kohlenstoffatomen bedeutet, wobei S und X in para-Stellung am Benzyl- oder Phenylrest stehen, als oberflächenaktive Mittel in diagnostischen Tests zur Aufhellung von Serum.

Solche Saccharide zeigen in einem mit Lipiden versetzten Testsystem eine über lange Zeit hinweg konstante Absorption.

Bevorzugte Saccharide sind Mono- und Oligosaccharide. Von den oligosacchariden sind wiederum die Di- und Trisaccharide bevorzugt. Von den Monosacchariden selbst sind Erythrose, Threose, Arabinose, Ribose, Xylose, Glucose, Mannose, Galactose und Fructose besonders bevorzugt. Von den Oligosacchariden, insbesondere von den Di- und Trisacchariden sind erfindungsgemäß solche bevorzugt, die Einheiten solcher Monosaccharide enthalten, wie z. B. Maltotriose. Unter den in den erfindungsgemäßen Verbindungen bzw. Mitteln enthaltenen Sacchariden sind auch entsprechende Abkömmlinge, wie Glucoronsäure oder Zuckeralkohole, wie Sorbit und Mannit zu verstehen. Für die in den erfindungsgemäßen Verbindungen enthaltenen Saccharideinheiten eignen sich auch die entsprechenden Deoxyverbindungen, wie Deoxyribose, Sorbose und Rhamnose. Ebenfalls sind erfindungsgemäß sowohl offene als auch ringförmige Zucker wie Furanosen und Pyranosen geeignet.

Besonders bevorzugte Disaccharide sind Saccharose, Maltose, Cellobiose und Trehalose. Ganz besonders bevorzugt sind jedoch Maltopyranosid, Galactopyranosid und Glucopyranosid.

Arylreste sind Phenyl- und Benzylreste. Von den $C_2$-$C_{20}$ Alkylresten sind $C_4$-$C_{18}$ und insbesondere $C_6$-$C_{12}$ Alkylreste bevorzugt. Die Alkylreste können verzweigt oder geradkettig sein und weisen ggf. auch ungesättigte Bindungen auf. Zweckmäßigerweise sind sie jedoch vollständig gesättigt. Ganz besonders bevorzugt sind Nonyl und Octyl und insbesondere Iso-Octylreste.

Die Arylreste tragen den Alkylsubstituenten in der para-Position zum Zuckerteil. Der Arylrest selbst ist mit dem

Zucker über glycosidische $\alpha$- und ß-Bindungen verknüpft.

Die Erfindung betrifft auch die erfindungsgemäße Verwendung in diagnostischen Tests zur Verringerung von Trübungen in insbesondere lipämischen Seren und zur Konstanthaltung einer solchen Trübung auf niedrigem Niveau.

Die Herstellung von mit Arylresten substituierten Sacchariden ist dem Fachmann bekannt und wird vorzugsweise nach dem in H.P. Kleine et al., Carbohydrate Research 142 (1985), 333-337 beschriebenen Verfahren durchgeführt. Danach werden die Saccharide zuerst, wie von C.S. Hudson und J.M. Johnson, J.Am. Chem.Soc. 37 (1915), 1270 und 1276 beschrieben, mit Natriumacetat und Essiganhydrid zu den entsprechenden Per-O-acetylierten Verbindungen umgesetzt. Die so erhaltenen Acetate werden in das entsprechende Bromid überführt und mit der gewünschten Arylverbindung in einer Phasentransfer-katalysierten Reaktion umgesetzt.

Ein weiteres Verfahren, mit dem die erfindungsgemäßen Substanzen erhalten werden können, ist von Kleine et al. in Carbohydrate Research 182 (1988), 307-312 beschrieben. Ein weiteres Verfahren zur Herstellung der erfindungsgemäßen Substanzen ist von J.Banoub und D.R. Bundle in Can.J.Chem. 57, 2085 (1979) beschrieben. Danach werden die vollständig acetylierten Zuckerverbindungen in Methylenchlorid bei tiefen Temperaturen mit der entsprechenden alkylsubstituierten Arylverbindung, insbesondere der Phenolverbindung, direkt zum gewünschten Produkt unter Katalyse mit einer Lewissäure (SnCl$_4$ ) umgesetzt.

Die Erfindung wird durch die folgenden Beispiele näher erläutert.

Beispiel 1

Nach dem von H.P. Kleine in Carbohydrate Research 142 (1985), 333-337 beschriebenen Verfahren wird i-Octylphenyl-glucopyranosid hergestellt.

Dazu werden 10 g 2,3,4,6-Tetra-O-acetyl-$\alpha$-D-glucopyranosylbromid in 100 ml Chloroform unter kräftigem Rühren gelöst. Die Lösung wird auf Rückfluß erhitzt und dann eine Lösung von 9,9 g Isooctylphenol 5,53 g Benzyltriethylammoniumbromid in 35 ml 1,25 mol/l NaOH-Lösung zugegeben. Nach drei Stunden läßt man den Ansatz abkühlen und gibt 100 ml Wasser zu. Die organische Phase wird abgetrennt, zweimal mit 1,25 mol/l NaOH ausgeschüttelt, getrocknet und anschließend das Lösungsmittel abgezogen. Man erhält ein gelbes Öl.

Die Abspaltung der Acetyl-Schutzgruppen erfolgt durch Auflösen und Ausrühren des Öls in einem Gemisch aus Methanol, Triethylamin und Wasser im Verhältnis 2:1:1. Das Lösungsmittel wird abdestilliert und das Produkt säulenchromatographisch gereinigt. Man erhält i-Octylphenyl-glucopyranosid in einer Ausbeute von 40 %.

In analoger Weise werden i-Octylphenyl-galactopyranosid und i-Octylphenyl-maltopyranosid (sowie die entsprechenden 4-Nonylphenyl-substituierten Analoga) hergestellt.

Beispiel 2:

Es wurden folgende Reagenzienlösungen hergestellt:
1,530 g Tris, 800 mg Natriumcholat und 1,50 g Magnesiumsulfat wurden in Wasser gelöst und mit (+) Weinsäure auf pH 7,15 eingestellt und mit bidestilliertem Wasser auf 200 ml aufgefüllt. Außerdem wurde 1 ml Intralipid® (erhältlich von Fa. Pfrimmer, Erlangen) (20 %ige Fettemulsion zur intravenösen Infusion) mit 27,57 ml bidestilliertem Wasser verdünnt. Auf diese Weise wurde eine 0,7 %ige Lipidlösung erhalten.

Für die Detergenzlösungen wurden jeweils 0,03 g Triton®-X100, 0,03 g des in Beispiel 1 erhaltenen Maltopyranosids, Galactopyranosids und Glucopyranosids mit 25 ml der obigen Reagenzlösung versetzt.

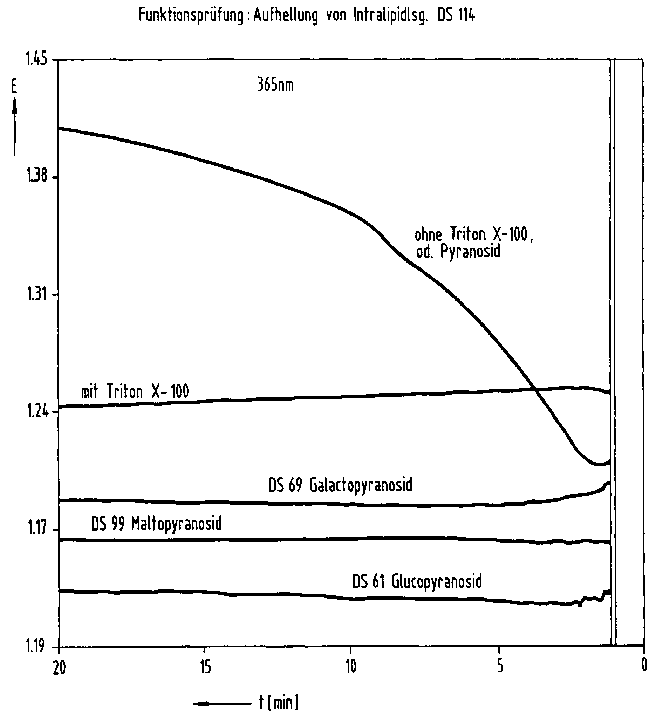

Zur Überprüfung der durch die Tenside hervorgerufenen Aufhellung der Lipidlösung wurden in einer Küvette (Schichtdicke 1 cm) 2,5 ml Reagenz/Detergenzlösung und 0,05 ml Lipidlösung pipettiert und gegen 2,5 ml Reagenz/Detergenzlösung mit 0,05 ml Zusatz von bidestilliertem Wasser als Leerwert in einem Photometer zeitabhängig bei einer Wellenlänge von 365 nm gemessen. Die Ergebnisse sind in Figur 1 dargestellt.

Daraus ist ersichtlich, daß ohne Zusatz eines Tensides bzw. Detergenz die Extinktion aufgrund der zunehmenden Trübung stetig zunimmt. Das Vergleichsdetergenz Triton®-X100 zeigt zwar eine zeitlich konstante Aufhellung, die jedoch bei einem wesentlich stärker absorbierenden Niveau stattfindet als bei den biologisch gut abbaubaren erfindungsgemäßen Sacchariden.

Beispiel 3:

Aufhellung von lipämischen Seren

Es wurde folgende Reagenslösung hergestellt:
1,530 g Tris, 800 mg Natriumcholat und 1,500 g Magnesiumsulfat wurden in Wasser gelöst und mit L(+)Weinsäure auf pH 7,15 eingestellt und mit bidestilliertem Wasser auf 200 ml aufgefüllt. Für die Detergenslösung wurden jeweils 0,03 g Triton® X-100 bzw. 0,03 g Maltopyranosid mit 25 ml der Reagenzlösung versetzt.

Die Aufhellwirkung der Tenside wurde an Humanseren mit verschiedenen Triglycerid-Gehalten getestet. Die verwendeten Seren enthielten ca. 200, 300, 500, 800, 1000 mg/dl Triglyceride.

Zur Überprüfung der durch die Tenside hervorgerufenen Aufhellung der Seren wurde in einer Küvette (Schichtdicke 1 cm) 2,5 ml Reagenz/Detergenzlösung und 0,05 ml Serum pipettiert und in einem Photometer zeitabhängig gemessen. Die Ergebnisse (optische Dichte in mE bei 365 nm) sind in Tabelle I dargestellt.

## Tabelle I

| Reagenzien | E (4 min) (mE) | E (10 min) (mE) | E (20 min) (mE) |
|---|---|---|---|
| **Serum 200 mg/dl** | | | |
| Triton X-100 | 167 | 166 | 165 |
| Maltopyranosid | 146 | 141 | 140 |
| Rgzlsg. o Tensid | 166 | 164 | 164 |
| | | | |
| **Serum 300 mg/dl** | | | |
| Triton X-100 | 288 | 286 | 285 |
| Maltopyranosid | 251 | 250 | 248 |
| Rgzlsg. o. Tensid | 300 | 298 | 299 |
| | | | |
| **Serum 500 mg/dl** | | | |
| Triton X-100 | 195 | 192 | 191 |
| Maltopyranosid | 170 | 168 | 166 |
| Rgzlsg. o. Tensid | 196 | 195 | 196 |
| | | | |
| **Serum 800 mg/dl** | | | |
| Triton X-100 | 593 | 588 | 584 |
| Maltopyranosid | 572 | 569 | 563 |
| Rgzlsg. o. Tensid | 651 | 652 | 658 |
| | | | |
| **Serum 1000 mg/dl** | | | |
| Triton X-100 | 553 | 546 | 543 |
| Maltopyranosid | 532 | 528 | 522 |
| Rgzlsg. o. Tensid | 606 | 613 | 618 |

**Patentansprüche**

1. Verwendung von Verbindungen der allgemeinen Formel

S - Ar - X

worin

Ar ein Benzyl- oder Phenylrest,
S ein mit Ar über eine α- oder ß-glycosidische Verbindung verknüpfter Saccharidrest und
X ein geradkettiger oder verzweigter Alkylrest mit 2 bis 20 Kohlenstoffatomen bedeutet, wobei S und X in para-Stellung am Benzyl- oder Phenylrest stehen, als oberflächenaktive Mittel in diagnostischen Tests zur Aufhellung von Serum.

2. Verwendung nach Anspruch 1,
**dadurch gekennzeichnet,**
daß das Saccharid ein Mono-, Di- oder Trisaccharid ist.

3. Verwendung nach Anspruch 1 oder 2,
**dadurch gekennzeichnet,**
daß das Saccharid Glucose, Mannose, Galactose, Fructose, Erythrose, Threose, Arabinose, Ribose und/oder Xylose ist oder solche Einheiten enthält.

4. Verwendung nach einem der Ansprüche 1 bis 3,
**dadurch gekennzeichnet,**
daß der Alkylrest ein $C_6$-$C_{12}$ Alkylrest ist.


**Claims**

1. Use of compounds having the general formula

S - Ar - X

wherein

Ar denotes a benzyl or phenyl residue
S denotes a saccharide residue linked to Ar via an α-glycosidic or β-glycosidic bond
X denotes a straight-chained or branched alkyl residue with 2 to 20 carbon atoms, wherein S and X are in the para position on the benzyl or phenyl residue, as surface active agents in diagnostic tests to clear serum.

2. Use as claimed in claim 1,
**wherein**
the saccharide is a monosaccharide, disaccharide or trisaccharide.

3. Use as claimed in claim 1 or 2,
**wherein**
the saccharide is glucose, mannose, galactose, fructose, erythrose, threose, arabinose, ribose and/or xylose or contains such units.

4. Use as claimed in one of the claims 1 to 3,
**wherein**
the alkyl residue is a $C_6$-$C_{12}$ alkyl residue.

**Revendications**

1. Utilisation de composés de formule générale

$$S - Ar - X$$

où

Ar représente un reste benzyle ou phényle,
S représente un reste saccharide lié à Ar par une liaison $\alpha$- ou $\beta$-glycosidique et
X représente un reste alkyle linéaire ou ramifié de 2 à 20 atomes de carbone, S et X étant en position para sur le reste benzyle ou phényle, comme agents tensioactifs dans des tests diagnostiques pour la clarification du sérum.

2. Utilisation selon la revendication 1, caractérisée en ce que le saccharide est un mono-, di- ou trisaccharide.

3. Utilisation selon la revendication 1 ou 2, caractérisée en ce que le saccharide est le glucose, le mannose, le galactose, le fructose, l'érythrose, le thréose, l'arabinose, le ribose et/ou le xylose ou contient de telles unités.

4. Utilisation selon l'une des revendications 1 à 3, caractérisée en ce que le reste alkyle est un reste alkyle en $C_6$-$C_{12}$.

Funktionsprüfung: Aufhellung von Intralipidlsg. DS 114

365nm

E

ohne Triton X-100,
od. Pyranosid

mit Triton X-100

DS 69 Galactopyranosid

DS 99 Maltopyranosid

DS 61 Glucopyranosid

t [min]